# EUROPEAN PATENT APPLICATION

(11) **EP 1 070 454 A1**
(43) Date of publication of application: **24.01.2001**
(21) Application number: 00902092.6
(22) Date of filing: 04.02.2000
(51) Int. Cl.: A01K 67/027, C12N 15/12, C12N 15/11, C12N 5/10, C12N 5/06, G01N 33/50, G01N 33/15

(54) **TRANSGENIC ANIMAL SHOWING VASCULAR TISSUE-SPECIFIC EXPRESSION OF ANGIOTENSIN II2 RECEPTOR**

(30) Priority: 05.02.1999 JP 2935499
(71) Applicant: SUNTORY LIMITED, Osaka-shi, Osaka 530-8203 (JP)
(72) Inventor: KURIHARA, Tatsuya, Mishima-gun, Osaka 618-0024 (JP); MATSUBARA, Hiroaki, Toyonaka-shi, Osaka 560-0002 (JP)
(74) Representative: Stein-Dräger, Christiane
(86) International application number: JP0000615
(87) International publication number: WO0045633

(57) **Abstract**

A transgenic animal and its progeny that vascular tissue-specifically express angiotensin II type 2 receptor; a method for producing said transgenic animal and its progeny; a recombinant gene for producing a transgenic animal, containing a promoter region derived from a protein gene expressed in vascular tissue and an angiotensin II type 2 receptor gene; a vascular tissue fragment or vascular cell derived from a transgenic animal that vascular tissue-specifically expresses angiotensin II type 2 receptor; a method for *in vivo* analyzing vascular physiological action and/or blood pressure-regulating action of angiotensin II type 2 receptor using said transgenic animal; and a method for *in vitro* analyzing vascular physiological action and/or blood pressure-regulating action of angiotensin II type 2 receptor using said vascular tissue fragment or vascular cell.

SEQUENCE LISTING
<110> Suntory Ltd.
<120> Transgenic Animal Having Angiotensin II Type 2 Receptor
<130> YCT-475
<160> 6

<210> 1
<211> 20
<212> Primer
<213> Artificial Sequence
<400> GATTATGGAG ATTAGAATTC

<210> 2
<211> 22
<212> Primer
<213> Artificial Sequence
<400> CTGGATCCGC TTCACAGGAT TC

<210> 3
<211> 17
<212> Primer
<213> Artificial Sequence
<400> TAACGGTCAG AGGATAG

<210> 4
<211> 17
<212> Primer
<213> Artificial Sequence
<400> TTCTGCTGGT GGCAGCA

<210> 5

<211> 21

<212> Primer

<213> Artificial Sequence

<400> GAGTGCATGC GGGAGCTGAG T

<210> 6

<211> 20

<212> Primer

<213> Artificial Sequence

<400> GGTGTCCATT TCTCTAAGAG

## Description

### FIELD OF THE INVENTION

The present invention relates to transgenic animals that are useful as animal models for studying physiological action, especially vascular physiological action and/or blood pressure-regulating action of angiotensin II type 2 receptor (AT2 receptor) and are also useful for explaining pharmaceutical effects of compounds acting on AT2 receptor and/or angiotensin II type 1 receptor (AT1 receptor).

### PRIOR ART

Angiotensin II (hereinafter sometimes referred to as AngII) is the most important in vivo vasopressor system for maintaining blood pressure and its action is mediated through specific receptor subtypes 1 (hereinafter referred to as AT1) and 2 (hereinafter referred to as AT2) (Matsubara, H. et al., Molecular insights Into angiotensin II type 1 and type 2 receptors: expression, signaling and physiological function and clinical application of its antagonists. Endocr. J. 1998; 45:137-150). AT1 receptor has the strongest *in vivo* vasoconstrictor effect mainly in adult vascular smooth muscle cells. A significant reduction of about 35 mmHg in average blood pressure was reported in AT1 receptor knockout mice (Sugaya, T. et al., Angiotensin II type 1a receptor-deficient mice with hypotension and hyperreninemia. J. Biol. Chem. 1995; 270:18719-18722).

On the other hand, AT2 receptor is a fetal-type receptor, which is systemically expressed mainly in mesenchymal systems during the fetal period, but the expression level rapidly decreases after birth. In adults, it is highly expressed in many brain sites including basal ganglia and in the uterus. The expression level is low in adult vessels, so that it has been thought to be less responsible for blood pressure control (Matsubara, H., Pathophysiological roles of angiotentin II type 2 receptor in cardiovascular and renal diseases. Circ. Res. 1998; 83:1182-1191).

In 1995, AT2 receptor knockout mice were produced (Ichiki, T. et al., Effects on blood pressure and exploratory behavior of mice lacking angiontensin II type-2 receptor. Nature. 1995; 377:748-750., Hein, L. et al., Behavioral and cardiovascular effects of disrupting the angiontensin II type-2 receptor gene in mice. Nature. 1995; 377:744-747), which showed an increased basal blood pressure as compared with wild-type counterparts and an excessive response to vasopressor action of AngII. This result suggests that AT2 receptor plays some role in regulating central/peripheral blood pressure, but its action mechanism has not been known because the expression of AT2 receptor could not be found in adult vascular systems.

In 1998, transgenic mice cardiac-specifically overexpressing AT2 receptor were produced (Masaki, H. et al., Cardiac-specific overexpression of angiontensin II AT2 receptor causes attenuated response to AT1 receptor-mediated pressor and chronotropic effects. J. Clini. Invets. 1998; 101:527-535), which were less sensitive to vasopressor or tachycardiac action of AngII so that AngII-mediated myocardial MAP kinase activation was suppressed, showing that AT2 receptor inhibits (vasopressor or tachycardiac) action of AT1 receptor. However, this action was cardiac but the blood pressure-regulating action of AT2 receptor on vascular systems was still unknown.

Intracellular signals of AT2 receptor have been known to activate tyrosine phosphatase to have cell growth inhibitory effect, but the relation with blood pressure-regulating action of AT2 receptor was unknown (Matsubara, H., Pathophysiological roles of angiotensin II type 2 receptor in cardiovascular and renal diseases. Circ. Res. 1998; 83:1182-1191).

The present invention provides a transgenic animal, which is useful as a model for studying vascular physiological action and/or blood pressure-regulating action of AT2 receptor so far involving much difficulty in expecting them. The reason why it was difficult to study the action of AT2 receptor seems to consist in that it is systemically expressed during the fetal period but the expression level rapidly decreases after birth to show a low expression level in adult vessels and that the expression cannot be found in adult vessels in animals. AT2 receptor is known to increase the expression in hypertrophied heart, infarcted heart and affected vascular endothelium, but its pathophysiological significance is wholly unknown.

### DISCLOSURE OF THE INVENTION

An object of the present invention is to provide an animal model for analyzing vascular physiological action and/or blood pressure-regulating action of AT2 receptor by producing a transgenic animal that vascular tissue-specifically expresses an AT2 receptor gene.

During analysis of the role of AT2 receptor by expressing an AT2 receptor gene in various tissues to attain the above object, we accomplished the present invention on the basis of the finding that AT2 receptor is expressed in vessels to show a blood pressure-regulating action when an animal is transfected with a recombinant gene containing AT2 receptor linked to a promoter of the vascular smooth muscle α-actin gene expressed in vascular smooth muscle.

Accordingly, the present invention provides a transgenic animal and Its progeny that vascular tissue-specifically express angiotensin II type 2 receptor.

The present invention preferably provides a transgenic animal and its progeny that vascular tissue-specifically express angiotensin II type 2 receptor, which are obtained by completing ontogenesis in a totipotent cell transfected with a recombinant gene for producing a transgenic animal containing a promoter region derived from a protein gene expressed in vascular tissue and an angiotensin II type 2 receptor gene.

The present invention also provides a method for producing said transgenic animal comprising the steps of inserting a recombinant gene for producing a transgenic animal containing a promoter region derived from a protein gene expressed in vascular tissue and an angiotensin II type 2 receptor gene into a totipotent cell, implanting said totipotent cell into a foster mother animal, and raising said foster mother animal to give birth to founder offspring expressing a desired character.

The present invention also provides a recombinant gene for producing a transgenic animal containing a promoter region derived from a protein gene expressed In vascular tissue and an angiotensin II type 2 receptor gene.

The present invention also provides a vascular tissue fragment or vascular cell derived from said transgenic animal.

The present invention also provides a method for *in vivo* analyzing vascular physiological action and/or blood pressure-regulating action of angiotensin II type 2 receptor using said transgenic animal.

The present Invention also provides a method for *in vitro* analyzing vascular physiological action and/or blood pressure-regulating action of angiotensin II type 2 receptor using said vascular tissue fragment or vascular cell.

### BRIEF DESCRIPTION OF THE DRAWINGS

FIG. 1 is a scheme showing construction of a recombinant gene for producing a transgenic animal.
FIG. 2 shows the results of PCR analysis of tail DNA of transgenic animals (electrophoretogram).
FIG. 3 shows the results of RT-PCR expression analysis in various tissues of a transgenic animal (electrophoretogram).

### THE MOST PREFERRED EMBODIMENTS OF THE INVENTION

The promoter region derived from a protein gene expressed in vascular tissue that can be used in the present invention is preferably a promoter region derived from a protein gene expressed in vascular smooth muscle. A promoter region derived from the vascular smooth muscle α-actin gene is especially preferred, which may be derived from mammals such as human or mouse.

The AT2 receptor gene that can be used in the present invention may be derived from mammals such as human or mouse, but not specifically limited thereto.

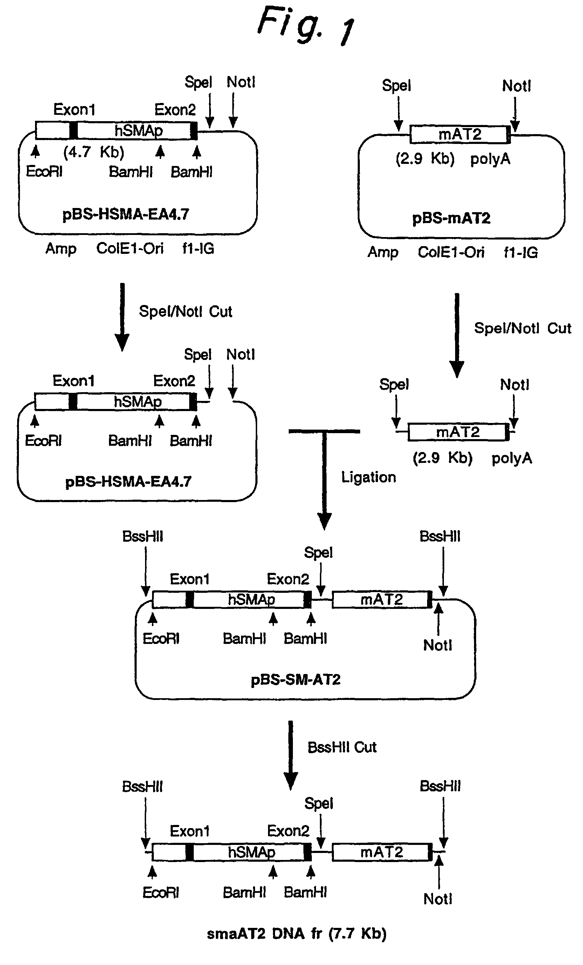

The recombinant gene for preparing a transgenic animal according to the present invention can be prepared by procedures known to those skilled in the art, as shown in Fig. 1, for example.

The present invention provides a transgenic animal that vascular tissue-specifically expresses AT2 receptor, which is a mammal and its progeny obtained by completing ontogenesis in a totipotent cell transfected with a recombinant gene for producing a transgenic animal containing an AT2 receptor gene linked downstream of a promoter region derived from a protein gene expressed in vascular tissue.

The target mammal may be technically any animal species other than human, but rodents are desirable because many inbred strains have been generated and manipulation techniques such as cultivation of fertilized eggs or *in vitro* fertilization have been well-established, among which mouse is especially preferred.

The totipotent cell used herein means a cell capable of potentially differentiating into all lineages of cells. In the case of mice, for example, the totipotent cell may be fertilized eggs and early embryos as well as cultured cells having pluripotency such as ES cells. Transfection of a DNA fragment into a cultured cell may be accomplished by using an electrostatic pulse, liposomes, calcium phosphate, etc. Physical injection of a DNA solution into a fertilized egg (microinjection) is especially preferred. The recombinant gene used in the method for producing a transgenic animal according to the present invention is desirably inserted during embryogenesis of a fertilized egg.

The cell transfected with DNA is implanted into a foster mother animal and said foster mother animal is raised to give birth to intended transgenic offspring (founder transgenic animal) expressing a desired character. The expression of the desired character in said animal can be identified by dissecting a part of the body (for example, the tail end) of said animal and isolating DNA in somatic cells to examine the existence of the transgene by PCR or Southern blotting or the like.

Progeny of transgenic animals according to the present invention can be produced by mating a founder transgenic animal with a wild-type heart to give F1 animals (progeny) and further mating those expressing a desired character among said F1 animals to give a homozygous population of F2 animals (progeny).

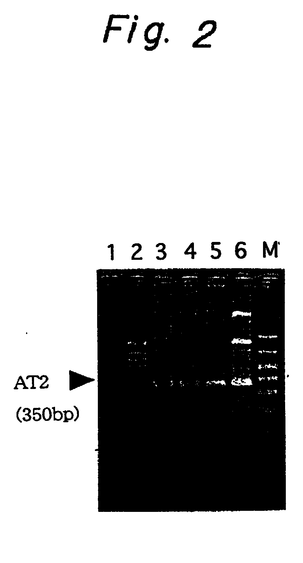

A founder transgenic animal and its progeny obtained by the present invention were assayed for the transgene by PCR using isolated and purified tail DNA and 3% agarose electrophoresis. As a result, a 350-bp AT2 receptor-specific band was observed In the founder transgenic animal and its progeny as shown in Fig. 2.

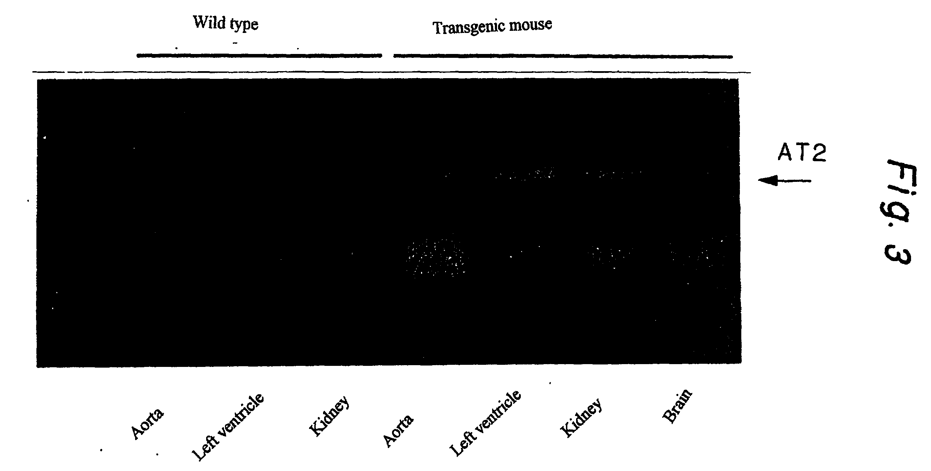

The expression analysis of the transgene was performed by RT-PCR in tissues of aorta, left ventricle, kidney and brain dissected from a wild-type animal and a transgenic animal (both adult). As shown in Fig. 3, no band for AT2 receptor could be detected in any organ (aorta, left ventricle, kidney) from the wild-type animal, but a 720-bp AT2 receptor-specific band was detected in each of the aorta, left ventricle, kidney and brain from the transgenic animal. All these organs have blood vessels, which confirmed that AT2 receptor is vascular tissue-specifically expressed.

The present invention also provides a method for *in vivo* analyzing vascular physiological action and/or blood pressure-regulating action of AT2 receptor using said transgenic animal. For example, AT2 receptor antagonists and agonists can be screened by administering AngII, an AngII antagonist or a candidate compound potentially having equivalent effects to the transgenic animal and observing the vascular physiological action and/or blood pressure-controlling action induced. Moreover, transgenic animals of the present invention can be used to evaluate the effects, especially side effects of an AT1 antagonist or AT1 agonist on AT2 receptor because currently used drugs known to act on AT1 receptor may also act on AT2 receptor depending on their receptor specificity. As shown in Example 4 below, the AT2 receptor expressed in transgenic animals of the present invention to which AngII was continuously administered suppresses vasopressor action of AngII.

The present invention also provides a vascular tissue fragment or a vascular cell derived from a transgenic animal of the present invention. A preferred vascular tissue fragment is a vascular smooth muscle tissue fragment such as an isolated aortic vascular smooth muscle tissue fragment. A preferred vascular cell is a vascular smooth muscle cell, for example. The present invention also provides a method for *in vitro* analyzing vascular physiological action and/or blood pressure-regulating action of AT2 receptor using said vascular tissue fragment or vascular cell. For example, isolated aorta of a transgenic animal can be used to determine the contractility of said isolated aorta after an agonist or antagonist of AT2 receptor or AT1 receptor or a candidate compound potentially having equivalent effects is added to a perfusion solution as described in Example 5 below. Alternatively, isolated aorta of a transgenic animal can be used to determine cGMP production or kininogenase activity in the isolated aorta after an agonist or antagonist of AT2 receptor or AT1 receptor or a candidate compound potentially having equivalent effects is added to a perfusion solution as described in Examples 6 and 7 below. Thus, screening for AT2 receptor antagonists and agonists as well as screening for AT1 receptor antagonists and agonists can be performed.

The following findings were obtained about vascular physiological action and/or blood pressure-regulating action of AT2 receptor using Isolated vascular tissue fragments of transgenic animals according to the present invention.
1) AT2 receptor reduced AngII-induced contraction of isolated vessels.
2) AT2 receptor significantly increased AngII-induced cGMP production and this increase was shown to be mediated by endothelium-derived vasodilator factor NO and bradykinin. This suggests that AT2 receptor has a hypotensive effect by activating a bradykinin/NO/cGMP production system known as a hypotensor system to reduce AngII-induced contraction of isolated vessels.
3) AT2 receptor was shown to participate in AngII-induced activation of kininogenase. This suggests that it has a hypotensive effect by increasing production of bradykinin from kininogen by kininogenase to reduce AngII-induced contraction of isolated vessels via a bradykinin/NO/cGMP production system known as a hypotensor system.

The following examples further illustrate the present invention, without limiting the same thereto. Various changes and modifications will be apparent to those skilled in the art and such changes and modifications are intended to also fall within the scope of the present invention.

### EXAMPLES

### Example 1: Preparation of a recombinant gene for producing a transgenic animal

The plasmid pBS-mAT2 containing the mouse AT2 receptor (Biophys. Biochem. Res. Commun. 1993; 197:393-399, ATCC S67465) was first subjected to double digestion with SpeI/NotI to give a cDNA fragment of 2.9 kb, as shown in Fig. 1.

Then, the plasmid pBS-HSMA-EA4.7 was provided, which was a gift from Dr. Takeshi Miwa, Genome Information Research Center of Osaka University in this example. The plasmid pBS-HSMA-EA4.7 can also be obtained by the following procedure.

A human vascular smooth muscle α-actin promoter is obtained by Long PCR using a human genome DNA library (HK1067j available from Clontech, CA, USA) as template DNA. On the basis of the primary sequence of a gene described in a prior document (Gene. 1991; 99:285-289), an upstream primer containing an EcoRI site (SMA-FE, -891, 5'-GATTATGGAGATTAGAATTC-3', 20mer; 20 µM) and a downstream primer containing a BamHI site (SMA-RB, +3828, 5'-CTGGATCCGCTTCACAGGATTC-3', 22mer, 20 µM) are prepared. Long PCR is performed with a TaKaRa Z-Taq kit to give a PCR product (4.7 kb). The DNA fragment is partially digested at both ends with EcoRI and BamHI to isolate and purify a DNA fragment of 4.7 kb, which is then ligated to an EcoRI/BamHI site of pBluescript II KS⁻ vector (Stratagene, La Jolla, CA, USA) to give the plasmid pBS-HSMA-EA4.7.

Then, the former cDNA fragment of 2.9 kb was ligated into a multiple cloning site of pBS-HSMA-EA4.7 after double digestion with unique restriction endonucleases SpeI and NotI and transformed into the E. coli JM109 strain (Toyobo Co., Ltd.) to give a plasmid pBS-SM-AT2.

For preparing a DNA solution for microinjection, the plasmid pBS-SM-AT2 was digested with BssHII to give a DNA fragment of 7.7 kb (smaAT2-DNA).

### Example 2: Production of transgenic animals

Mice for collecting fertilized eggs to be transfected with smaAT2-DNA (egg collecting mice) were C57BL/6J pure strain mice purchased from CLEA Japan, Inc. Females at 8 weeks of age or older were superovulated and mated with males at 8 weeks of age or older to collect many fertilized eggs, which were transferred to M2 medium and cultured in a 5% carbon dioxide incubator at 37°C.

Then, 2 pl of smaAT2-DNA solution was injected into the male pronucleus of each of said fertilized eggs by microinjection using a DNA injection pipette.

The fertilized eggs injected with smaAT2-DNA solution were transferred to M16 medium and cultured overnight in a 5% carbon dioxide incubator at 37°C.

Female mice for pregnancy, delivery and nursing of offspring from the fertilized eggs injected with smaAT2-DNA solution (foster mother mice) and male mice mated with the females were Inbred ICR mice purchased from CLEA Japan, Inc. Vasoligated male mice at 8 weeks of age or older were mated with female mice at 8 weeks of age or older, among which those showing a vaginal plug were used as foster mothers. Each foster mother was intraperitoneally injected with an anesthetic at 0.01 ml/g body weight containing Nembutal (Dainabot Co., Ltd., sodium pentobarbital, 50 mg/ml) diluted to 12% in a diluent (a mixed solution of 20 ml propylene glycol, 10 ml ethanol and 70 ml sterilized water) to expose the left and right oviducts by surgery.

The fertilized eggs injected with smaAT2-DNA solution were cultured overnight and those having developed into 2-cell embryos were collected and 10-15 of them were inserted into each oviduct, after which the incised site was sutured.

Foster mothers were raised for 3 weeks and if they delivered, the tail of each offspring was dissected at about 1 cm 5 weeks after birth to isolate and purify chromosomal DNA using Easy-DNA Kit (Invitrogen).

This tail DNA was tested for the presence of the transgene by PCR.

The mice in which the presence of the transgene was identified were reared as founder transgenic mice up to the age of 7 weeks and then naturally mated with wild-type C57BL/6J at 7 weeks of age or older to give transgenic progeny.

The following results were obtained in this example. The smaAT2-DNA solution was injected into 1182 eggs identified as fertilized eggs among 2878 eggs collected from the total of 179 egg collecting mice C57BL/6J. On the following day, 858 eggs (73%) developed into 2-cell embryos, which were implanted into the oviducts of a total of 33 foster mothers. Twenty foster mothers became pregnant and gave birth to a total of 85 offspring (10%). An assay for the transgene by PCR in the tail DNA showed that a total of 7 founder transgenic mice (1 male, 6 females) (8%) were obtained. These founder transgenic mice were naturally mated with wild type C57BL/6J to give progeny. 6 founder transgenic mice except 1 female mouse transmitted the transgene to both male and female progeny.

### Example 3: Gene analysis of transgenic animals

The transgene was identified by PCR using isolated and purified tail DNA. Thirty cycles of a 3-stage reaction involving 93°C for 1 minute, 50°C for 1 minute and 72°C for 1 minute were performed in 50 µl of a reaction solution containing 1 µl of each tail DNA sample as template DNA, 4 µl of a mixed solution of 2.5 mM each dGTP, dATP, dTTP, and dCTP, 0.5 µl of 5 u/µl TaKaRa Taq (Takara Shuzo Co., Ltd.), 5 µl of 10 x PCR Buffer (buffer annexed to TaKaRa Tag available from Takara Shuzo Co., Ltd.), 2 µl of an upstream primer (EaTg-F, 5'-TAACGGTCAGAGGATAG-3', 17mer, 20 µM), and 2 µl of a downstream primer (AT2-R, 5'-TTCTGCTGGTGGCAGCA-3', 17mer, 20 µM).

After PCR reaction, a 10 µl aliquot was assayed by 3% agarose electrophoresis.

Fig 2 shows the results of this experiment indicating that a founder transgenic mouse and its progeny contain the introduced DNA in their chromosomes. M represents a DNA molecular weight marker indicating electrophoretic positions at 1000, 700, 500 + 525, 400, 300, 200, 100 and 50 bp from the top down. The samples used as template DNA were as follows: lane 1, distilled water; lane 2, a wild-type mouse; lane 3, a founder transgenic mouse; lane 4, a progeny of the founder transgenic mouse; lane 5, another progeny of the founder transgenic mouse; lane 6, the introduced DNA as a positive control. A 350-bp AT2 receptor-specific band was found in the founder transgenic mouse and its progeny.

The expression analysis of the transgene was performed by RT-PCR (Fig. 3). Tissues of aorta, left ventricle, kidney and brain were dissected from wild-type and transgenic mice to isolate and purify total RNA by a standard procedure. RT-PCR was performed on 15 µg RNA using an upstream primer (AT2-F3, 5'-GAGTGCATGCGGGAGCTGAGT-3', 21mer, 20 µM) and a downstream primer (AT2-R2, 5'-GGTGTCCATTTCTCTAAGAG-3', 20mer, 20 µM). As a result, no band could be detected from the wild-type mouse, but a 720-bp AT2 receptor-specific band was detected in each organ from the transgenic mouse.

### Example 4: Blood pressure change after continuous administration of angiotensin II

Change of average awaking blood pressure induced by chronic administration of angiotensin II (AngII) was compared between transgenic and wild-type mice. AngII (0.7 mg/day/kg) was chronically administered via an osmotic pump embedded in the back of each mouse, and the blood pressure of the mouse was measured at the tail by the tail-cuff method (Table 1). The wild-type mice showed an increase of 38 mmHg in average blood pressure after 2 weeks, while the mice that vascular smooth muscle-specifically express AT2 receptor (AT2-TG) showed no blood pressure change. This shows that the AT2 receptor expressed in the transgenic mice suppressed vasopressor action of AngII.

**Table 1**

| Average blood pressure under administration of angiotensin II (mmHg) | | | | |
|---|---|---|---|---|
| | Day 0 | Day 4 | Day 7 | Day 14 |
| Wild-type mice | 83±2.7 | 94±2.1* | 114±2.8* | 121±3.2* |
| AT2-TG mice | 85±1.8 | 84±2.7 | 87±2.1 | 89±2.6 |

| | | | | |
|---|---|---|---|---|
| The values represent means ± SE of average blood pressure; * P< 0.05. | | | | |

### Example 5: Angiotensin II-induced contraction of isolated aorta

AngII-induced contraction of isolated aortic vessels was compared between transgenic and wild-type mice. After abdominal incision of each mouse, the abdominal aorta was isolated and cut into rings, which were then suspended under a constant tension of 0.8 g in a myograph. Change of vascular tension after addition of AngII was recorded under perfusion with Tyrode's solution (137 mmol/L NaCl, 2.7 mmol/L KCl, 1.8 mmol/L CaCl₂, 1.1 mmol/L MgCl₂, 0.42 mmol/L NaH₂PO₄, 12 mmol/L NaHCO₃ and 5.7 mmol/L glucose, pH 7.4) (Table 2). AngII-induced contraction of isolated aorta increased by about 24% in AT2-TG mice in the presence of an AT2 receptor inhibitor (PD123319, 1-[[4-(dimethylamino)-3-methylphenyl]methyl]-5-(diphenylacetyl)-4,5,6,7-tetrahydro-1*H*-imidazo[4,5-*c*]pyridine-6-carboxylic acid ditrifluoroacetate monohydrate available from PARKE-DAVIS, Ann Arbor, Michigan) as compared with the contraction in its absence.

However, no contraction change was observed in the presence of PD123319 in wild-type mice. This shows that the AT2 receptor expressed in the transgenic mice reduces AngII-induced contraction of isolated vessels.

**Table 2**

| Angiotensin II-induced vasoconstriction (% of control) | | |
|---|---|---|
| | -PD123319 | +PD123319 |
| Wile-type mice | 100±0.7 | 98±1.1 |
| AT2-TG mice | 100±0.2 | 124±2.1* |

| | | |
|---|---|---|
| The values represent means ± SE of average blood pressure; * P< 0.01. | | |

### Example 6: Angiotensin II-induced cGMP production in isolated aorta

Then, AngII-induced cGMP production in isolated aortic vessels was compared between transgenic and wild-type mice in order to examine whether or not vasodilator action of AngII is mediated by cGMP. After abdominal incision of each mouse, the abdominal aorta was isolated and the aortic extract homogenized by sonication in ethanol was dried in nitrogen and treated with an acetylation reagent (acetic anhydride : triethylamine = 1:2) and then assayed for cGMP level by radioimmunoassay using an anti cGMP antibody (Table 3). The amount of cGMP produced in isolated aorta did not change in the wild-type mice in the presence of an AngII + AT1 antagonist CV11974 (AT2 receptor stimulation, (±)-1-(cyclohexyloxy-carbonyloxy)ethyl-2-ethoxy-1-[[2'-(1-H-tetrazol-5-yl)-bipbenyl-4-yl]methyl]-1H-benzimidazole-7- carboxylate available from Takeda Chemical Industries, Ltd.), but it increased by 105% in vessels of AT2-TG mice. This increase was completely inhibited by endothelium removal, an NO synthase inhibitor (L-NAME) and a bradykinin receptor antagonist (icatibant). This shows that the AT2 receptor expressed in the transgenic mice significantly increases cGNP production and that this increase is mediated by endothelium-derived vasodilator factor NO and bradykinin. Namely, this suggests that it has a hypotensive effect by activating a bradykinin/NO/cGMP production system known as a hypotensor to reduce AngII-induced contraction of isolated vessels as shown in Example 5.

**Table 3**

| Angiotensin II-induced cGMP production in isolated aorta (fmol/gwt) | | | | |
|---|---|---|---|---|
| | AngII | AngII + CV11974 | AngII + CV11974 + L-NAME | AngII + CV11974 + icatibant |
| Wild-type mice | 328±14 | 372±18 | 350±28 | 310±25 |
| AT2-TG mice | 330±41 | 676±27* | 298±21 | 316±28 |

| | | | | |
|---|---|---|---|---|
| The values represent means ± SE of average blood pressure; * P< 0.01. | | | | |

### Example 7: Angiotensin II-induced kininogenase activity in isolated aorta

Then, the activity of kininogenase, which is an enzyme for producing bradykinin from kinonogen, was compared between transgenic and wild-type mice in isolated aortic vessels. After abdominal incision of each mouse, the abdominal aorta was isolated and the aortic extract homogenized in the presence of 0.1 M phosphate buffer (0.2% bacitracin, 3 mM O-phenanthroline, 30 mM EDTA-Na₂) containing a protease inhibitor was dried in nitrogen under 90% ethanol. The dried extract was solubilized in a buffer containing an angiotensin-converting enzyme inhibitor and tested for kininogenase activity by radioimmunoassay of kallikrein produced from bovine kininogen as a substrate (Table 4). Kininogenase activity in isolated aorta did not change in the wild-type mice in the presence of an AngII + AT1 antagonist CV11974 (AT2 receptor stimulation), but it increased by 150% in vessels of AT2-TG mice. This increase was completely inhibited by endothelium removal, an NO synthase inhibitor (L-NAME) and a bradykinin receptor antagonist (icatibant).

This shows that the AT2 receptor expressed in the transgenic mice participates in AngII-induced activation of kininogenase. Namely, this suggests that it has a hypotensive effect by increasing production of bradykinin from kininogen by kininogenase to reduce AngII-induced contraction of isolated vessels as shown in Example 5 via a bradykinin/NO/cGMP production system known as a hypotensor system.

**Table 4**

| Angiotensin II-induced kininogenase activity in isolated aorta (ng kinin/mg/h) | | | | |
|---|---|---|---|---|
| | AngII | AngII + CV11974 | AngII + CV11974 + L-NAME | AngII + CV11974 + icatibant |
| Wild-type mice | 0.68±0.11 | 0.75±0.15 | 0.66±0.15 | 0.65±0.17 |
| AT2-TG mice | 0.76±0.07 | 1.9±0.18* | 0.71±0.09 | 0.81±0.17 |

| | | | | |
|---|---|---|---|---|
| The values represent means ± SE of average blood pressure; * P< 0.001. | | | | |

### INDUSTRIAL APPLICABILITY

Transgenic animals expressing AT2 receptor in vascular tissue can be obtained by the technical procedures described above. Such transgenic animals are useful as models for studying the mechanism of vascular physiological action and/or blood pressure-regulating action of AT2 receptor and are effectively applicable in the research field such as an assay system for antagonists and agonists of AT2 receptor and/or AT1 receptor.

## Claims

1. A transgenic animal and its progeny that vascular tissue-specifically express angiotensin II type 2 receptor.

2. The transgenic animal and its progeny that vascular tissue-specifically express angiotensin II type 2 receptor according to Claim 1, which are obtained by completing ontogenesis in a totipotent cell transfected with a recombinant gene for producing a transgenic animal containing a promoter region derived from a protein gene expressed in vascular tissue and an angiotensin II type 2 receptor gene.

3. The transgenic animal and its progeny according to Claim 1 or 2, which are rodents.

4. The transgenic animal and its progeny according to Claim 3, which are mice.

5. A method for producing the transgenic animal according to Claim 1 comprising the steps of inserting a recombinant gene for producing a transgenic animal containing a promoter region derived from a protein gene expressed in vascular tissue and an angiotensin II type 2 receptor gene into a totipotent cell, implanting said totipotent cell into a foster mother animal, and raising said foster mother animal to give birth to founder offspring expressing a desired character.

6. The method according to Claim 5 wherein the promoter region is derived from a vascular smooth muscle protein gene.

7. The method according to Claim 6 wherein the promoter region is derived from the vascular smooth muscle α-actin gene.

8. A recombinant gene for producing a transgenic animal, comprising a promoter region derived from a protein gene expressed in vascular tissue and an angiotensin II type 2 receptor gene.

9. The recombinant gene according to Claim 8 wherein the promoter region is derived from a vascular smooth muscle protein gene.

10. The recombinant gene according to Claim 8 wherein the promoter region is derived from the vascular smooth muscle α-actin gene.

11. The vascular tissue fragment or vascular cell derived from the transgenic animal according to Claim 1.

12. The vascular tissue fragment according to Claim 11, which is a vascular smooth muscle tissue fragment.

13. The vascular cell according to Claim 11, which is a vascular smooth muscle cell.

14. A method for *in vivo* analyzing vascular physiological action and/or blood pressure-regulating action of angiotensin II type 2 receptor using the transgenic animal according to Claim 1.

15. A method for *in vitro* analyzing vascular physiological action and/or blood pressure-regulating action of angiotensin II type 2 receptor using the vascular tissue fragment or vascular cell according to Claim 11.
